# EUROPÄISCHE PATENTSCHRIFT

(11) **EP 0 776 650 B1**
(45) Veröffentlichungstag und Bekanntmachung des Hinweises auf die Patenterteilung: **09.07.2003**
(21) Anmeldenummer: 96115507.4
(22) Anmeldetag: 27.09.1996
(51) Int. Cl.: A61K 6/083

(54) **Dentaladhäsive**
Dental adhesive
Adhésif dentaire

(30) Priorität: 30.11.1995 DE 19544673
(43) Veröffentlichungstag der Anmeldung: 04.06.1997
(73) Patentinhaber: Heraeus Kulzer GmbH & Co.KG, 63450 Hanau (DE)
(72) Erfinder: Podszun, Wolfgang, Dr., 51061 Köln (DE); Finger, Werner, Prof. Dr., 41469 Neuss (DE); Heiliger, Ludger, Dr., 51373 Leverkusen (DE)
(74) Vertreter: Kühn, Hans-Christian

(56) Entgegenhaltungen:
- EP-A- 0 206 074
- EP-A- 0 290 133
- EP-A- 0 546 648
- WO-A-93/12760

## Beschreibung

Die Erfindung betrifft eine Zubereitung zur Verwendung als Adhäsivkomponente für die Behandlung der Zahnhartsubstanz.

Ein besonders gravierendes Problem in der konservierenden Zahnheilkunde besteht in der dauerhaften, randspaltfreien Verklebung von Kunststoff-Füllungsmaterialien mit der Zahnhartsubstanz (Dentin und Schmelz). Im Dentalbereich werden aushärtbare Materialien als Füllungsaterialien bei Zahnreparaturen verwendet. Als aushärtbare Materialien werden im allgemeinen Füllungsmaterialien auf Acrylatbasis bevorzugt, die durch radikalische Polymerisation ausgehärtet werden. Ein Nachteil dieser Materialien besteht darin, dass sie während der Aushärtung schrumpfen und so zur Randspaltbildung beitragen. Die Kunststoff-Füllungen haben den zusätzlichen Nachteil, dass sie schlecht am Dentin haften bleiben.

Um die Bindung an die Zahnhartsubstanz zu verbessern, kann man sogenannte Dentaladhäsive einsetzen. Dabei werden Dentaladhäsive bevorzugt, die sowohl am Dentin als auch am Schmelz gute Haftwerte ergeben. Wirksame Formulierungen enthalten im allgemeinen eine Vielzahl von Komponenten. So wird in DE-A-38 28 170 ein Beschichtungsmittel für kollagenhaltige Materialien beschrieben, das aus
a) Aldehyd,
b) einem wasserlöslichen Monomer mit aktivem Wasserstoff,
c) einem wasserunlöslichen Monomer mit zwei oder mehreren polymerisierbaren Doppelbindungen,
d) einem Fotoaktivator,
e) Wasser,
f) einem Löslichkeitsvermittler und/oder Dispergator und
g) bekannten Zusätzen
besteht.

In DE-A-41 05 550 wird eine Zubereitung aus
a) einem Formamidoalkylmethacrylat,
b) einem (Meth)acrylsäureester, der Vernetzungen ausbilden kann,
c) einem Lösungsmittel,
d) gegebenenfalls weiteren Zusätzen,
e, einer Säure und
f) gegebenenfalls einem Dispergator
als Dentaladhäsiv beschrieben.

EP 206 074 A offenbart Zusammensetzungen mit neuen, cycloaliphatischen Methacrylaten. Als Reaktiwerdünner können Comonomere zugefügt werden, darunter GDMA = Glycerindimeth acrylat (Seite 11, Zeilen 20 - 25). Die Verbindungen finden ggf. zusammen mit Füllstoffen Verwendung als Zahnfüllmaterialien und Versiegter (Sealants), enthalten aber keine Lösungsmittel. Sie können redox-, UV- oder lichtgehärtet werden.

Es wurde nun eine Zubereitung gefunden, die wenige Komponenten enthält, sehr einfach angewendet werden kann und sehr hohe Haftwerte an Schmelz und Dentin ermöglicht.

Die erfindungsgemäße Zubereitung ist dadurch gekennzeichnet, dass sie aus
a) 30 - 70 Gew.-% Glycerindi(meth)acrylat,
b) 70 - 30 Gew.-% eines flüchtigen, mit Wasser mischbaren Lösungsmittels,
c) 0,01 - 2,5 Gew.-% Fotoaktivator und gegebenenfalls
d) 0 - 40 Gew.-% an sich bekannter Zusätze aus der Gruppe der Coaktivatoren, Stabilisatoren, Inhibitoren und Lichtschutzmittel
besteht, mit dem Vorbehalt, daß die Summe der Gewichtsanteile 100% ergibt.

Glycerindi(meth)acrylate im Rahmen der vorliegenden Erfindung sind die Diester von Glycerin mit Acrylsäure und Methacrylsäure. Beispielhaft seien die nachfolgenden Verbindungen aufgeführt.

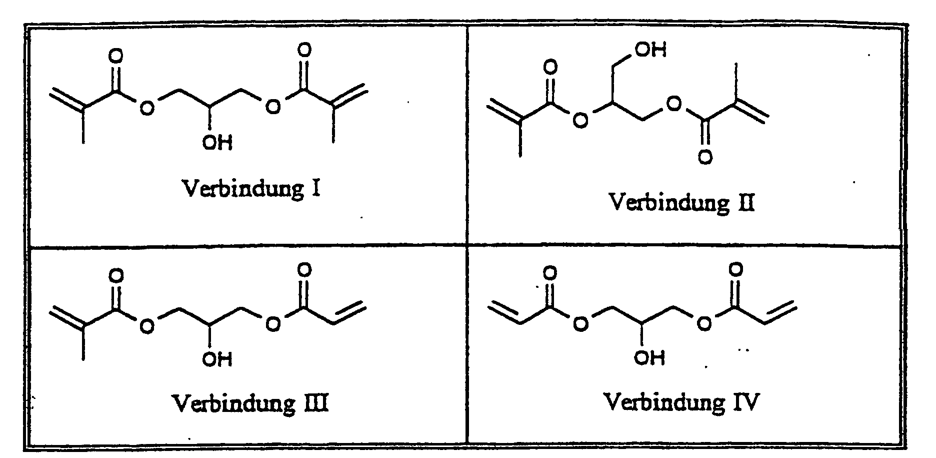

Natürlich können auch Mischungen verschiedener Glycerindi(meth)acrylate, insbesondere auch Isomerengemische aus 1,2-Glycerindi(meth)acrylaten und 1,3-Glycerindi(meth)acrylaten, eingesetzt werden.

Flüchtige, mit Wasser mischbare Lösungsmittel sind vor allem solche mit einem Dampfdruck von mindestens 100 Torr bei Raumtemperatur. Bevorzugt sind aliphatische Alkohole mit ein bis vier C-Atomen, Aceton, 1,4-Dioxan und Tetrahydrofuran. Besonders bevorzugt sind Aceton und Ethylalkohol.

Fotoaktivatoren beziehungsweise -initiatoren im Rahmen der vorliegenden Erfindung sind Radikalbildner, die unter der Einwirkung von Licht, beispielsweise UV-Licht, sichtbarem Licht oder Laserlicht, eine radikalische Polymerisation auslösen.

Diese Fotopolymerisationsinitiatoren sind an sich aus der Literatur bekannt. Vorzugsweise handelt es sich um Mono- oder Dicarbonylverbindungen, wie Benzophenon, Benzoin und dessen Derivate, insbesondere Benzoinmethylether, Benzil und Benzilderivate und andere Dicarbonylverbindungen, wie Diacetyl, 2,3-Pentandion und a-Diketoderivate des Norbomans und substituierter Norbomane, Metallcarbonyle, wie Pentacarbonylmangan, oder Chinone, wie 9,10-Phenanthrenchinon und Naphthochinon. Besonders bevorzugt ist Campherchinon.

Die erfindungsgemäße Zubereitung enthält im allgemeinen 0,01 bis 2,5 Gew.-%, bevorzugt 0,1 bis 0,5 Gew.-%, des Fotoaktivators, bezogen auf die erfindungsgemäße Zubereitung.

Es kann vorteilhaft sein, der erfindungsgemäßen Zubereitung Coaktivatoren zuzusetzen, die die Polymerisationsreaktion beschleunigen. Bekannte Beschleuniger sind beispielsweise Amine, wie p-Toluidin, Dimethyl-p-toluidin, Trialkylamine, wie Trihexylamin, Polyamine, wie N,N,N',N'-Tetraalkylalkylendiamine, Barbitursäure und Dialkylbarbitursäuren. Besonders bevorzugt sind Dimethylaminobenzolsulfonamide entsprechend DE-A-31 35 113.

Die Coaktivatoren werden im allgemeinen in einer Menge von 0,02 bis 4 Gew.-%, bevorzugt 0,2 bis 1 Gew.-%, bezogen auf die erfindungsgemäße Zubereitung, eingesetzt.

Es wurde nun gefunden, dass die an sich sehr hohen Haftwerte an Dentin durch einen Anteil an Füllstoff noch weiter gesteigert werden können. Geeignete Füllstoffe sind beispielsweise Bergkristall, Kristobalit, Quarzglas, hochdisperse Kieselsäuren, Aluminiumoxid und Glaskeramiken. Die mittlere Teilchengröße der Füllstoffe liegt im allgemeinen im Bereich von 10 - 2000 nm, vorzugsweise im Bereich von 10 - 100 nm. Besonders gut geeignete Füllstoffe sind hochdisperse Kieselsäuren, die beispielsweise durch Flammhydrolyse gewonnen werden können.

Eine bevorzugte erfindungsgemäße Zubereitung ist dadurch gekennzeichnet, dass sie aus
a) 30 - 70 Gew.-% Glycerindi(meth)acrylat,
b) 70 - 30 Gew.-% eines flüchtigen, mit Wasser mischbaren Lösungsmittels,
c) 0,01 - 2,5 Gew.-% Fotoaktivator,
d1) 5 - 20 Gew.-% Füllstoff und gegebenenfalls
d2) 0 - 25 Gew.-% an sich bekannter Zusätze aus der Gruppe der Coaktivatoren, Stabilisatoren, Inhibitoren und Lichtschutzmittel
besteht mit dem Vorbehalt, daß die Summe der Gewichtsanteile 100% ergibt.

Die Füllstoffe werden vorzugsweise vorbehandelt, beispielsweise mit Silanisierungsmitteln aus Organosilicium-Verbindungen (Progress in Organic Coatings 11, 297 - 308 (1983). Ein bevorzugtes Silanisierungsmittel ist 3-Methacryloyloxypropyl-trimethoxysilan.

Die erfindungsgemäße Zubereitung kann in einfacher Weise durch Mischen der einzelnen Komponenten hergestellt werden. Sie wird als Adhäsivkomponente zur Behandlung von Zahnhartsubstanz verwendet.

In einer besonderen Ausführungsform konditioniert man die Zahnhartsubstanz vor der Behandlung mit der erfindungsgemäßen Zubereitung mit einer Konditionierungsflüssigkeit, die einen pH-Wert im Bereich von 0,1 bis 3,5 aufweist. Diese Konditionierungsflüssigkeit enthält im allgemeinen Säuren mit einem pKs-Wert kleiner als 5 und gegebenenfalls eine amphotere Aminoverbindung mit einem pKs-Wert im Bereich von 9,0 bis 10,6 und einem pKb-Wert im Bereich von 11,5 bis 12,5. Folgende Säuren können z. B. in der Konditionierungsflüssigkeit enthalten sein: Phosphorsäure, Salpetersäure, Brenztraubensäure, Zitronensäure, Oxalsäure, Ethylendiamintetraessigsäure, Essigsäure, Weinsäure, Äpfelsäure. Weiterhin kann die Konditionierungsflüssigkeit Stoffe aus der Gruppe der Polyethylenglykole und Metallhydroxide enthalten. Insbesondere können die oben aufgeführten mehrbasigen Säuren auch als teilweise Metallsalze eingesetzt werden, solange freie Säurefunktionen verbleiben. Bevorzugt wird die Behandlung mit wäßriger Phosphorsäure. Geeignete Konzentrationen der Phosphorsäure sind 10 - 60 Gew. -%, vorzugsweise 20 - 40 Gew.-%. Die Konditionierungsflüssigkeit kann zur Einstellung einer geeigneten Konsistenz Verdickungsmittel, z. B. Kieselsäure, enthalten.

Die Anwendung der erfindungsgemäßen Zubereitung kann beispielsweise wie folgt durchgeführt werden:

Bei einer Zahnreparatur trägt man nach einer mechanischen Reinigung der Zahnoberfläche zuerst die Konditionierungsflüssigkeit auf, lässt eine kurze Zeit (beispielsweise 60 Sekunden) einwirken, spült die Zahnoberfläche mit Wasser und trocknet sie. Dann trägt man die erfindungsgmäße Zubereitung, beispielsweise mit einem kleinen Pinsel, in einer oder mehreren dünnen Schichten auf, trocknet im Luftstrom und bestrahlt mit einer handelsüblichen Polymerisationslampe. Danach trägt man das eigentliche Füllungsmaterial, beispielsweise ein im Dentalbereich übliches Kunststoff-Füllungsmaterial, auf.

Zur näheren Erläuterung werden im folgenden einige Beispiele für die erfindungsgemäße Zubereitung (Beispiele 1 - 4) und die Prüfung ihrer Wirksamkeit durch Bestimmung der Scherbindungsfestigkeit von Kunststoff-Füllungen auf damit vorbehandeltem Dentin und Schmelz (Beispiel 5) beschrieben.

### Beispiel 1

Die folgende Zubereitung wird durch intensives Vermischen der Bestandteile erzeugt.

| Beispiel 1 | |
|---|---|
| Glycerindimedimethacrylat (Verbindung I) | 5,0 g |
| Aceton | 5,0 g |
| Campherchinon | 20 mg |
| Diallylsulfonamid* | 50 mg |

### Beispiel 2

Die folgende Zubereitung wid durch intensives Vermischen der Bestandteile erzeugt.

| Beispiel 2 | |
|---|---|
| Glycerindimethacrylat (Verbindung I) | 5,0 g |
| Ethanol | 5,0 g |
| Campherchinon | 20 mg |
| Diallylsulfonamid* | 50 mg |

### Beispiel 3

Die folgenden Zubereitungen werden durch intensives Vermischen der Bestandteile erzeugt.

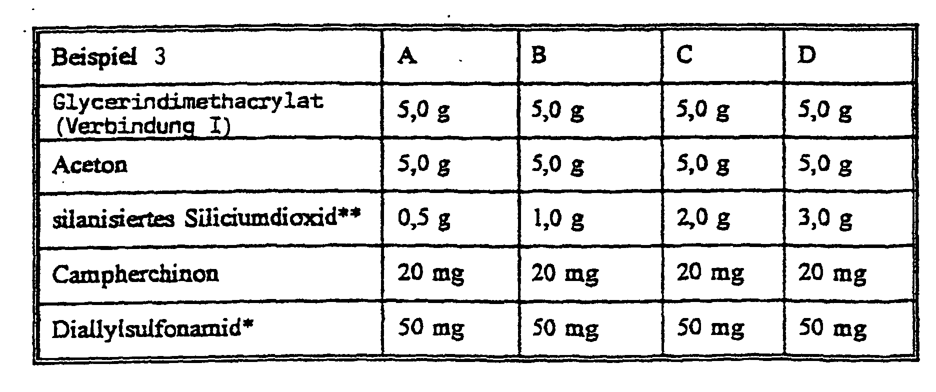

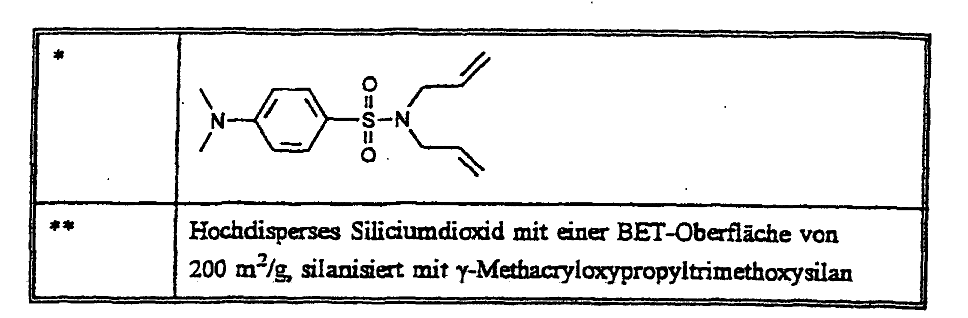

### Beispiel 4

### Bestimmung der Scherbindungsfestigkeit an Dentin

Die Wirksamkeit der in den Beispielen 1 - 3 beschriebenen Zubereitungen als Dentinadhäsive wird geprüft durch Bestimmung der Scherbindungsfestigkeit auf Dentin. Es werden menschliche Zähne benutzt, die für max. drei Monate nach der Extraktion in 1 % Chloraminlösung aufbewahrt waren. Vor der Verwendung im Bindungstest werden die Zähne nach sorgfältiger Reinigung unter fließendem Wasser für mindestens drei und höchstens zehn Tage in physiologischer Kochsalzlösung gelagert. Am Tage vor der Verwendung im Bindungstest werden die Zähne einzeln auf einer Approximalseite liegend mit Expoxidharz (Lekutherm® X20, Härter T3) in zylindrische Gummiformen von 25 mm Durchmesser und 12 mm Höhe eingebettet. Die Zähne werden durch Nassschleifen mit SiC-Papieren der Kömungen 240, 320, 400 und schließlich 600 soweit beschliffen, dass eine ausreichend große schmelznahe Dentinfläche zur Anbindung eines Kunststoffzylinders mit 3,5 mm Durchmesser freiliegt. Nach Abspülen mit entionisiertem Wasser und Trocknung im Luftstrom wird 30 Sekunden lang das Konditionierungsmittel Gluma® CPS Gel (20 % H3PO4, Bayer) mit einem Wattepellet unter reibender Bewegung aufgetragen, mit Wasser sorgfältig abgespült und durch Abtupfen mit Zellstoff oberflächlich von Wasser befreit (feuchte Technik). Auf die konditionierte Dentinfläche werden die Zubereitungen aus den Beispielen 1 - 3mit einem Pinsel in drei Schichten aufgetragen, im Druckluftstrom getrocknet und mit dem Lichtgerät TRANSLUX® CL (Kulzer) 20 Sekunden lang bestrahlt. Die so vorbehandelte Probe wird dann mittels einer Einspannvorrichtung unter einer zweigeteilten zylindrischen Teflonform (3,5 mm Durchmesser, 1 mm Höhe) festgeklemmt. Danach wird das Kunststoff-Füllungsmaterial PEKAFILL® (U) mit einer Spritze in die Teflonform eingefüllt, mit einem 02-undurchlässigen Strip abgedeckt und mit dem Lichtgerät TRANSLUX® CL 60 Sekunden lang bestrahlt. Unmittelbar anschließend wird die Teflonform abgenommen und die zylindrische Probe für 24 Stunden in 37° C warmem Wasser gelagert bis zur Einleitung der Scherbelastung. Dazu wird die zylindrische Probe in einer Universalprüfmaschine mit Hilfe eines Druckstempels parallel zu und dicht an der angeschliffenen Zahnoberfläche bei einer Geschwindigkeit von 1 mm/Minute bis zur Trennung des Zylinders vom Zahn belastet. Die Scherbindungsfestigkeit ist der Quotient aus Bruchkraft und Bindungsareal und wird jeweils an 5 Proben bestimmt und als deren Mittelwert in der Tabelle angegeben.

### Bestimmung der Scherbindungsfestigkeit an Schmelz

Für die Bestimmung der Scherbindungsfestigkeit an mit den in den Beispielen 1 - 3 beschriebenen Zubereitungen behandeltem Schmelz werden extrahierte menschliche Zähne mit intakter labialer Schmelzfläche in Epoxidharz eingebettet und mit nassem SiC-Papier der Körnung 240 bis 600 angeschliffen, um eine plane periphere Schmelzoberfläche freizulegen. Auf die Schmelzoberfläche wird das Konditionierungsmittel Gluma® CPS Gel aufgetragen, das nach 30 Sekunden sorgfältig mit entionisiertem Wasser abgespült wird. Die Trocknung erfolgt nur oberflächlich mit einem schwachen Druckluftstrahl, bis die behandelte Oberfläche kreidig weiß erscheint. Alle weiteren Arbeitsschritte sind identisch mit den vorstehend beschriebenen zur Bestimmung der Scherbindungsfestigkeit an Dentin. Die Werte für die Scherbindungsfestigkeit an Schmelz werden in der Tabelle angegeben.

| Zubereitung | Scherbindungsfestigkeit an Dentin | Scherbindungsfestigkeit an Schmelz |
|---|---|---|
| Beispiel 1 | 14,5 MPa | 34,7±1,7 MPa |
| Beispiel 2 | 18,6 MPa | 32,5±5,4 MPa |
| Beispiel 3A | 14,7 MPa | 33,6±4,6 MPa |
| Beispiel 3B | 19,0 MPa | 29,5±3,3 MPa |
| Beispiel 3C | 14,5 MPa | 34,0±2,9 MPa |
| Beispiel 3D | 11,0 MPa | 17,9±4,1 MPa |

## Patentansprüche

1. Zubereitung zur Verwendung als Adhäsivkomponente für die Behandlung der Zahnhartsubstanz, **dadurch gekennzeichnet, dass** sie aus
a) 30 - 70 Gew.-% Glycerindi(meth)acrylat,
b) 70 - 30 Gew.-% eines flüchtigen, mit Wasser mischbaren Lösungsmittels,
c) 0,01 - 2,5 Gew.-% Fotoaktivator und gegebenenfalls
d) 0 - 40 Gew.-% an sich bekannter Zusätze aus der Gruppe der Coaktivatoren, Füllstoffe, Stabilisatoren, Inhibitoren, Lichtschutzmittel besteht,
mit dem Vorbehalt, daß die Summe der Gewichtsanteile 100% ergibt.

2. Zubereitung nach Anspruch 1, **dadurch gekennzeichnet, dass** sie aus
a) 30 - 70 Gew.-% Glycerindi(meth)acrylat,
b) 70 - 30 Gew.-% eines flüchtigen, mit Wasser mischbaren Lösungsmittels,
c) 0,01 - 2,5 Gew.-% Fotoaktivator,
d1) 5 - 20 Gew.-% Füllstoff und gegebenenfalls
d2) 0 - 25 Gew.-% an sich bekannter Zusätze aus der Gruppe der Coaktivatoren, Stabilisatoren, Inhibitoren, Lichtschutzmittel
besteht, mit dem Vorbehalt, daß die Summe der Gewichtsanteile 100% ergibt.

3. Zubereitung nach Anspruch 2, **dadurch gekennzeichnet, dass** als Füllstoff hochdisperse Kieselsäure mit einer mittleren Teilchengröße von 5 - 2000 nm, vorzugsweise von 10 - 50 nm, eingesetzt wird.

4. Verfahren zur Herstellung der Zubereitung nach einem der Ansprüche 1 bis 3, **dadurch gekennzeichnet, dass** die Komponenten a) bis d) miteinander vermischt werden.

## Claims

1. Formulation for use as an adhesive component for the treatment of hard tooth substance, **characterized in that** it consists of
a) 30 - 70% by weight of glyceryl di(meth)acrylate,
b) 70 - 30% by weight of a volatile, water-miscible solvent,
c) 0.01 - 2.5% by weight of photoactivator and optionally
d) 0 - 40% by weight of additives known per se and from the group consisting of the coactivators, fillers, stabilizers, inhibitors and light stabilizers,
with the proviso that the sum of the amounts by weight is 100%.

2. Formulation according to Claim 1, **characterized in that** it consists of
a) 30 - 70% by weight of glyceryl di(meth)acrylate,
b) 70 - 30% by weight of a volatile, water-miscible solvent,
c) 0.01 - 2.5% by weight of photoactivator,
d1) 5 - 20% by weight of filler and optionally
d2) 0 - 25% by weight of additives known per se and from the group consisting of the coactivators, stabilizers, inhibitors and light stabilizers,
with the proviso that the sum of the amounts by weight is 100%.

3. Formulation according to Claim 2, **characterized in that** the filler used is colloidal silica having a mean particle size of 5 - 2000 nm, preferably of 10 - 50 nm.

4. Process for the preparation of the formulation according to any of Claims 1 to 3, **characterized in that** the components a) to d) are mixed with one another.

## Revendications

1. Préparation pour l'utilisation comme composant adhésif pour le traitement d'une substance dure dentaire, **caractérisée en ce qu'**elle comprend
a) 30 - 70 % en poids de di(méth)acrylate de glycérine,
b) 70 - 30 % en poids d'un solvant volatil, miscible avec l'eau,
c) 0,01 -2,5 % en poids d'un photoactivateur et éventuellement
d) 0 - 40 % en poids d'un additif connu en soi du groupe des coactivateurs, des charges, des stabilisants, des inhibiteurs, des agents de protection contre la lumière, avec la condition que la somme des quantités en poids soit de 100 %.

2. Préparation selon la revendication 1, **caractérisée en ce qu'**elle comprend
a) 30 - 70 % en poids de di(méth)acrylate de glycérine,
b) 70 - 30 % en poids d'un solvant volatil, miscible avec l'eau,
c) 0,01 - 2,5 % en poids d'un photoactivateur,
d1) 5 - 20 % en poids d'une charge et éventuellement
d2) 0 - 25 % en poids d'un additif connu en soi du groupe des coactivateurs, des stabilisants, des inhibiteurs, des agents de protection contre la lumière, avec la condition que la somme des quantités en poids soit de 100%.

3. Préparation selon la revendication 2, **caractérisée en ce qu'**on utilise comme charge de l'acide silicique finement dispersé avec une taille de particules moyenne de 5 - 2000 nm, de préférence 10 - 50 nm.

4. Procédé de fabrication de la préparation selon l'une des revendications 1 à 3, caractérisé en de que les composants a) à d) sont mélangés les uns avec les autres.
